# EUROPEAN PATENT APPLICATION

(11) **EP 2 163 194 A2**
(43) Date of publication of application: **17.03.2010**
(21) Application number: 09425327.5
(22) Date of filing: 20.08.2009
(51) Int. Cl.: A61B 5/05

(54) **System of impedentiometric evaluation on teleassistance for physics body condition**

(30) Priority: 09.09.2008 IT AV20080005 U
(71) Applicant: Vicinanza, Aldo, 84098 Pontecagnano Faiano (SA) (IT)
(72) Inventor: Vicinanza, Aldo, 84098 Pontecagnano Faiano (SA) (IT)

(57) **Abstract**

This wire system is comprised of a bi-polar electric cable in which at one end four adhesive probes with sensors are connected and at the other end an USB-type device is attached. The system is linked to a server containing the data that will be used by the client. The driver with this application is available on a server website. Following a web connection the user can insert the USB-type device in a free port on the PC and place the adhesive sensors with probes on the left foot and the left hand.

The client application will guide the user in performing measurements for him and others and send the anthropometric and impedance data obtained to an online server. Once the server has received the data from the user, it sees to its elaboration and the results are then delivered to the user. This finding is an industrial and agricultural product.

## Description

Scientific research, new systems studies and new technologies aim increasingly towards the improvement of the quality of life by means of a more healthier and correct nutrition. Doctors, nutritionists and anyone who is concerned for his welfare is in accord with the fact that correct eating habits not only maintain the organism young and energetic, but also help in the prevention of important diseases.

For this reason, industrial technology has made available new instruments and analysing systems that can allow any individual, without any medical background, the possibility to carry out measurements and obtain immediate scientific support to evaluate the state of physical welfare and consequently make changes in the diet.

The measurement of body impedance is one of the most commonly used and reliable techniques for the determination of body composition. It is based on the scientific fact that water is a good conductor of electric current and that fat is a perfect insulator. As the lean body mass (Fat Free Mass -FFM) consists primarily of water, determining the organism's water content, it is easily possible to work out the values of FFM and FAT MASS (FAT).

For this purpose an instrument called a body impedance assessment meter or bioimpedance assessment meter is used. When it is connected by means of electrodes to the user, it measures the resistance that the body offers to the passage of a weak current at high frequency. From the value of the body impedance, one can work out the body-water content, lean mass, fat mass, and the basal metabolism of the subject under examination. The full range of biochemical processes in the human organism (nutrition, assimilation, respiration, digestion, absorption and excretion) is the task of the metabolism.

Water balance is tightly correlated to metabolism. A state of well-balanced hydration is the first requisite for the metabolism to function correctly. The bioimpedance test was developed with the aim to detect human body impedance through the passage of an electric current The best professional bioimpedance assessment meters are those that directly measure the impedance components, namely resistence (R), reactance (X) and phase angle (PA). It is of great importance that the instruments evaluate resistance and reactance separately, so that for example, by a vector analysis it is possible to see and get an idea of the state of hydration and cell composition of the subject under examination by simply looking at a spot on a graph.

There are two types of body impedance assessment meters available on the market: monofrequency and multifrequency meters. In both cases however, the presence in loco of an operator is indispensable because of the particular physical and mechanical characteristics of these instruments.

In fact, to date, users that would like to have these measurements taken have the inconvenience of having to go to centres that operate in this sector and this often entails considerable economic costs.

Noteworthy is the need to allow anyone the possibility to carry out a body bioimpedance analysis in a simple, fast and safe manner so as to verify the state of personal welfare, as well as that of friends and colleagues, and take the necessary measures if needed to correct bad eating habits or integrate with vitamin and mineral supplements in case of deficiencies.

This finding, a system to measure bioimpedance from home or where it is more convenient for the user would be extremely convenient on the market, as to date, a simple and functional system does not exist.

Moreover, self-analysis will allow for constant monitoring of the state of physiological welfare of large areas of population.

The new model consists of a bi-polar electric cable that according to the law UNEL 35023 has the minimum section of the cable not inferior to 0,25 mm²/A (4/A mm²), four electrodes made up of adhesive probes with sensors and an USB-type device.

It is made available to the user already assembled It is a wire system with a bi-polar electric cable in which at one end the adhesive probes with sensors are connected and at the other end the USB-type device is attached.

This system is then linked to a server that provides an online service for acquisition, elaboration, and delivery of results. Drivers with this application can be installed on the user's PC from a server website designed for this.

After connecting to the web, the user can insert the USB-type device to a free port on (his/her) computer and attach the adhesive probes with sensors on the left foot and left hand. The client application will guide the user in performing the impedance measurement using current intensity ≤ 900µA regulated by a closed-loop current injector, voltage ≤ I Vpp intercepted by a "balun" type detector, 50KHz ±1% sinusoidal frequency controlled by a microprocessor.
Impedance data, together with other parameters acquired by the probes can be visualized and this information can then be sent to the server for online service, for elaboration and delivery of results to the user.

The requisites for the patent can be attributed to the novelty represented by this new model, not already included in the technique, it confers particular efficacy and commodity in its application to the resolution of the problem regarding human body or animal bioimpedance measurements and to the acquisition and elaboration of emerging data.

The inventive process involved the elaboration of a combination of parts and conformations and dispositions that were not obvious to an expert in the field

The industrial application of the finding is given by its construction and use in general in the industrial and agricultural fields.

In accordance with the law, the finding is not contrary to public order, good custom or laws that forbid production and in its application makes use of non-invasive technology.

For this finding, it is requested to obtain a patent as a utility model according to the Legislative Decree 10.02.2005, n. 30 ("Codice dei Diritti di Proprietà Industriale"), published on the Official Journal (Gazzetta Ufficiale) S.O. n. 52 of 04.03.2005, it consists of a model aimed at conferring particular efficacy or commodity in its application and in its use of electric cables and probes with sensors for the detection of impedance data following the passage of a weak current at high frequency and an USB-type device. This new combination of parts will allow the acquisition of impedance data in an individual.

Since the finding has been described and represented in a developmental and geometric form, developed as a demonstration but not limited in its essential characteristics, it is clear that it can be subject to modifications and adjustments according to industrial and commercial needs and used together with other systems and means without defeating its original purpose. Therefore it must be clear that in the request to obtain the patent, any equivalent application of these concepts and any equivalent product developed and/or operating according to one or more of the characteristics included in the following claims must be indicated.

## Claims

1. A wire system made up of a bi-polar electric cable in which at one end four adhesive probes with sensors are connected and at the other ends an USB-type device is attached.

2. A bi-polar electric cable which according to the law UNEL 35023 has the minimum section of the cable not inferior to 0,25 mm²/A (4/A mm²), four electrodes made up of adhesive probes with sensors and an USB-type device.
It is made available to the user already assembled
The system is linked to a server that provides an online service for the acquisition, elaboration, and delivery of results to the user.
Drivers with this application that can be installed on the user's battery-power-supplied laptop eliminating the need for industrial or domestic electric power networks, are available on a server website designed for this.
After connecting to the web, the user can insert the USB-type device in a free port on (his/her) computer and place the adhesive probes with sensors on the left foot and left hand. The client application will guide the user in performing the impedance measurement using current intensity ≤ 900µA regulated by a closed-loop current injector, voltage ≤ 1Vpp intercepted by a "balun" type detector, 50KHz±1% sinusoidal frequency controlled by a microprocessor. Impedance data, together with other parameters acquired by the probes can be visualized and this information can then be sent to the server for online service for elaboration and delivery of results to the user.

3. A tele-assisted impedance evaluation system for self-analysis of physical conditions carried out by placing on the left hand and left foot four electrodes with probes and sensors.
With this finding at hand, the user can place the electrodes, insert the USB-type device in a free USB port of a battery-power-supplied computer and connect to the web. By accessing to the designated website, the driver with the application that will guide the user in performing the various impedance measurements for himself and others and furnish anthropometric data needed for the elaboration of physiological functions of the subject under examination, can be downloaded.

4. The finding as to the above claims is made of the following materials: metal, plastic, rubber, synthetic resins or natural products and in any other suitable material;

5. The finding as to the above claims **characterized by** all that is claimed, described and represented in the present request to obtain an industrial patent.
